# EUROPEAN PATENT APPLICATION

(11) **EP 4 276 847 A1**
(43) Date of publication of application: **15.11.2023**
(21) Application number: 22173256.3
(22) Date of filing: 13.05.2022
(51) Int. Cl.: G16H 40/20

(54) **DEFINING A TIMESTAMP FOR A TARGET MEDICAL EVENT**

(71) Applicant: Koninklijke Philips N.V., 5656 AG Eindhoven (NL)
(72) Inventor: RODRIGUES DE MOURA LEITAO, Ana Jorge, Eindhoven (NL); CHATTERJEA, Supriyo, Eindhoven (NL); DIKIC , Marko, Eindhoven (NL); TOLHUIZEN, Ludovicus Marinus Gerardus Maria, Eindhoven (NL); FURNICA, Alexander Sebastian, Eindhoven (NL); BOESING, Josee, Eindhoven (NL); SYNNATSCHKE, Anne, Eindhoven (NL); BEEKERS, Pim, Eindhoven (NL)
(74) Representative: Philips Intellectual Property & Standards

(57) **Abstract**

A mechanism for defining or determining a timestamp for a target medical event. One or more location-derived timestamps are determined from event location data, which identifies one or more potential locations for the target medical event and entity location data, which identifies locations and times of relevant entities for the target medical event. The location-derived timestamps are then processed, using a rules-based technique, to produce the timestamp.

## Description

### FIELD OF THE INVENTION

The present invention relates to the field of medical events, and in particular, to determining or defining at time at which a medical event occurs.

### BACKGROUND OF THE INVENTION

In clinical environments, the time at which a medical event occurs is commonly recorded as a timestamp. A medical event may represent any clinically important or useful event, e.g., arrival at the clinical environment, performance of a scan, assessment by a clinician, occurrence of a sign or symptom and so on.

There is a need for any recorded timestamps to be accurate. A large number of medical decisions, e.g., diagnostic or treatment decisions, are made on the basis of such timestamps. By way of example, different treatment options may be preferred depending on a time lapsed since the onset of a particular symptom. As another example, a change in subject condition between temporally proximate assessments of the subject is of greater clinical concern than a change in subject condition between temporally distant assessments, as the former could show sudden deterioration.

There is therefore a benefit to ensuring that timestamps are accurate, and more accurate timestamps for past medical events would credibly assist a clinician in treating and/or assessing the condition of a subject or patient.

### SUMMARY OF THE INVENTION

The invention is defined by the claims.

According to examples in accordance with an aspect of the invention, there is provided a computer-implemented method for defining a timestamp for a target medical event for a subject. The computer-implemented method comprises: obtaining event location data identifying one or more potential locations for the target medical event; obtaining entity location data comprising, for one or more relevant entities, one or more location data entries, each location data entry identifying a location of the relevant entity and a location timestamp, wherein each relevant entity is an entity associated with the target medical event; processing the event location data and the entity location data to establish one or more location-derived timestamps, each location-derived timestamp identifying a time at which a respective one of the one or more relevant entities is at or near one of the one or more potential locations for the target medical event identified by the event location data; and processing at least the one or more location-derived timestamps using a rule-based technique to define the timestamp for the target medical event.

Embodiments provides a mechanism for determining or defining a timestamp for a target medical event. The defined timestamp can effectively act as the recorded timestamp for the target medical event. One or more location-derived timestamps for the target medical event are determined, and used to define the timestamp. This approach recognizes that the location of relevant entities for the target medical event defines or dictates the time(s) at which the target medical event can occur. By using the location-derived timestamps to establish the timestamp for the target medical event, an accuracy in the defined timestamp for the target medical event is increased (e.g., compared to manually-recorded timestamps alone).

The rule-based technique provides a system for quickly and efficiently defining the timestamp based on available or relevant timestamps for the target medical event. The proposed method may be implemented by a processing system, e.g., a processing system that forms part of a larger health information system.

The benefit of more accurate timestamps for medical events has been previously explained, namely to aid a clinician or further processing system in making or assisting the making of a more appropriate clinical decision for a subject.

Preferably, the computer-implemented method comprises storing the defined timestamp for the target medical event in an electronic medical record for the subject, e.g., to accompany an entry in the electronic medical records for the target medical event. The defined timestamp may, for instance, overwrite or otherwise replace an existing timestamp in the electronic medical record (e.g., a manually-recorded timestamp) for the target medical event. In this way, a recorded timestamp for the target medical event in the electronic medical record can be set/modified to align with the defined timestamp.

Other uses for the defined timestamp are also envisaged. For instance, the method may output the defined timestamp to a further processing system. Thus, labelled more generally, the method may comprise a step of outputting the defined timestamp.

In the context of the present invention, a "potential location" is a location at which it is expected or anticipated that the target medical event may or should take place. For instance, if the target medical event is a CT scan, the potential location may include any room or area used for performing a CT scan. Other examples will be apparent to the skilled person.

Generally, a timestamp represents a value or measure of time, e.g., according to the ISO 8601 standard, although other approaches can be adopted. For instance, a location timestamp identifies a time at which the corresponding relevant entity is at a particular location.

The use of location-derived timestamps helps create a more accurate characterization of the time(s) taken for subjects to move through various stages of a care pathway than simply relying on manually-input data. This is mainly because location-derived timestamps can be automatically generated when a change in the subject's location corresponds to a change in the treatment stage. Automated timestamp generation, using the approach adopted by the present disclosure, also frees up the time of care providers since it reduces the overhead of having to manually log information.

The one or more relevant entities may include the subject of the target medical event. This embodiment recognizes that the presence of the subject is essential for many medical events, such that the subject location-derived timestamp for the subject provides important information on when the target medical event took place.

Accordingly, the one or more location-derived timestamps preferably comprises a subject location-derived timestamp indicating a time at which the subject is at or near the one or more potential locations for the target medical event identified by the event location data. The subject location-derived timestamp may be the earliest time that the subject is at or near the one or more potential locations for that particular visit to/near the potential location(s).

In some examples, the step of processing, using a rule-based technique, comprises defining the timestamp for the target medical event to be no earlier than the subject location-derived timestamp. This recognizes that the presence of the subject is essential for many medical events, such that the target medical event cannot take place before the subject is in the potential location.

The one or more relevant entities may include one or more clinicians or non-subject individuals involved in the medical event. A non-subject individual is an individual who is not the subject, but is involved in the medical event, e.g., a porter, family member or assistant for the individual.

The one or more location-derived timestamps may comprise one or more non-subject individual location-derived timestamps, each indicating a time at which a respective clinician or non-subject individual is at or near the one or more potential locations for the target medical event identified by the event location data. The non-subject location-derived timestamp may be the earliest time that the clinician or non-subject individual is at or near the one or more potential locations for that particular visit to/near the potential location(s).

In some examples, the step of processing, using a rule-based technique, comprises defining the timestamp for the target medical event to be no earlier than any of the one or more non-subject individual location-derived timestamps. This approach recognizes that the occurrence of a target medical event cannot occur before all relevant entities for the target medical event are at or near a potential location. In this way, the accuracy of the defined timestamp is improved.

In some examples, the one or more relevant entities includes one or more pieces of equipment for the target medical event.

The one or more location-derived timestamps may comprise one or more equipment location-derived timestamps, each indicating a time at which a respective piece of equipment is at or near the one or more potential locations for the target medical event identified by the event location data. Each equipment location-derived timestamp may be the earliest time that the piece of equipment is at or near the one or more potential locations for that particular visit to/near the potential location(s).

The step of processing, using a rule-based technique, may comprise defining the timestamp for the target medical event to be no earlier than any of the one or more equipment location-derived timestamps. This approach recognizes that the occurrence of a target medical event cannot occur before all relevant entities for the target medical event are at or near a potential location. In this way, the accuracy of the defined timestamp is improved.

In some examples, the method further comprises: obtaining relevant entity data identifying, for a plurality of medical events, one or more entities associated with each medical event; and processing the relevant entity data to identify, for the target medical event, the one or more relevant entities for the target medical event. This provides a mechanism for establishing which entities (amongst a plurality of possible entities) are relevant for the particular target medical event. Different target medical events will have different relevant entities. This approach ensures that the most appropriate entities for the target medical event are identified.

The method may further comprise, obtaining, if available, a manually recorded timestamp for the target medical event, wherein, the step of processing, using a rule-based technique, further processes at least the manually recorded timestamp, if available, to define the timestamp for the target medical event. Manually recorded timestamps may contain valuable information for defining the timestamp of the target medical event. In particular, a manually-recorded timestamp may act as a "backup" if a timestamp cannot be accurately determined from other information.

In some examples, the method further comprises obtaining event workflow data defining the expected sequence for a plurality of medical events for the subject, the plurality of medical events including at least the target medical event; and obtaining workflow timestamp data defining at least one timestamp for each of the plurality of medical events in the event workflow data. In such examples, the step of processing, using a rule-based technique, further processes the event workflow data and workflow timestamp data to define the timestamp for the target medical event.

This approach recognizes that the timings of other medical events that surround, e.g., precede or follow, the target medical event will influence, affect or indicate the true timestamp for the target medical event. For instance, if the target medical event is "arrival at hospital", it is not possible for this target medical event to occur after any other medical event at the hospital.

The event workflow data defines a sequence of events, including the target medical event, that facilitate identification of where/when the timestamp for the target medical event should fall. This facilitates improved accuracy in identifying and defining the timestamp for the target medical event.

In some examples, the workflow timestamp data comprises, for each of one or more automatically recordable events in the plurality of events, an automatically recorded timestamp for the automatically recordable event.

The step of processing, using a rule-based technique, may comprise defining the timestamp for the target medical event to be no later than the automatically recorded timestamp for any medical event later in the expected sequence, defined by the event workflow data, than the target medical event.

This approach recognizes that automatically generated timestamps are typically highly accurate. An accuracy for a timestamp for a target medical event can therefore be improved by preventing the timestamp for the target medical event from falling after an automatically recorded timestamp for a later medical event.

The step of processing, using a rule-based technique, may comprise defining the timestamp for the target medical event to be no earlier than the automatically recorded timestamp for any medical event earlier in the expected sequence, defined by the event workflow data, than the target medical event. An accuracy for a timestamp for a target medical event can therefore be improved by preventing the timestamp for the target medical event from falling before an automatically recorded timestamp for an earlier medical event.

In some examples, the method may comprise a step of outputting the defined timestamp for the target medical event. Outputting the defined timestamp for the target medical event may comprise setting and/or modifying a recorded timestamp for the target medical event stored by an electronic medical record to align with the defined timestamp for the target medical event. Advantages of this approach have been previously described.

Proposed methods may be iteratively repeated for a plurality of medical events, e.g., each medical event in a sequence of medical events.

There is also proposed a computer program product comprising computer program code means which, when executed on a computing device having a processing system, cause the processing system to perform all of the steps of the method according to any herein claimed method.

There is also proposed a processing system for defining a timestamp for a target medical event for a subject. The processing system is configured to: obtain event location data identifying one or more potential locations for the target medical event; obtain entity location data comprising, for one or more relevant entities, one or more location data entries, each location data entry identifying a location of the relevant entity and a location timestamp, wherein each relevant entity is an entity associated with the target medical event; process the event location data and the entity location data to establish one or more location-derived timestamps, each location-derived timestamp identifying a time at which a respective one of the one or more relevant entities is at or near one of the one or more potential locations for the target medical event identified by the event location data; and process at least the one or more location-derived timestamps using a rule-based technique to define the timestamp for the target medical event.

The processing system may be further configured to output, e.g., at an output interface, the defined timestamp for the target medical event.

The processing system may be configured to output the defined timestamp by setting and/or modifying a recorded timestamp for the target medical event stored by an electronic medical record to align with the defined timestamp for the target medical event. Thus, the processing system may be configured to control an electronic medical record, e.g., stored by an EMR database, to set/modify a recorded timestamp for the target medical event, e.g., to be equal to the defined target timestamp.

The processing system may be modified to perform any herein disclosed method, and vice versa. Any advantages of the computer-implemented method according to the present invention analogously and similarly apply to the system and computer program herein disclosed.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates an approach according to an embodiment;
Figure 2 is a flowchart illustrating a method according to an embodiment; and
Figure 3 is a flowchart illustrating a method for use in an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The disclosure provides a mechanism for defining or determining a timestamp for a target medical event. One or more location-derived timestamps are determined from event location data, which identifies one or more potential locations for the target medical event and entity location data, which identifies locations and times of relevant entities for the target medical event. The location-derived timestamps are then processed, using a rules-based technique, to produce the timestamp.

Embodiments are based on the realization that the location or "real-time location" of relevant entities for a medical event define or otherwise indicate the time at which that medical event occurs. By using location-derived timestamps for a medical event, a more accurate estimate or determination of the timestamp for the target medical event can be achieved.

Proposed approaches can be employed in any clinical environment in which a medical event takes place, and for which information on the timing of the medical events would be beneficial. Examples include hospitals, clinics, birthing centers, pharmacies and so on.

In the context of the present invention, a timestamp or medical event that is "earlier" than another timestamp or medical event takes place temporally before the said other timestamp or medical event. Similarly, a timestamp or medical event that is "later" than another timestamp or medical event takes place temporally after the said other timestamp or medical event.

An underlying recognition of the present disclosure is that timestamps in electronic medical records (EMRs) are often inaccurate. This is because it is common for such timestamps to be manually recorded or entered by clinicians or staff retrospectively, sometimes long after the corresponding medical events have taken place. For instance, it is common for a clinician to perform a batch entry of timestamps (e.g., towards the end of their shift), which can introduce error, mistakes and/or noise into the timestamps.

It has been herein recognized that it is possible to correct at least some of these manually entered timestamps and/or introduce missing timestamps using timestamps derived from automated or computer-implemented means. In particular, location tracking of at least the subject and/or other relevant entities can be used to define an earliest and/or latest possible opportunity a particular medical event took place, setting a lower and/or upper bound for a range of possible timestamps. This information can be used to modify existing timestamps and/or establish more accurate timestamps that more closely resemble the true or "ground-truth" timestamp(s) for the subject.

The present disclosure thereby provides an approach or mechanism for estimating or establishing a timestamp for a target medical event.

A medical event is any event of a medical workflow that may have an impact on the clinical success or health outcome of a subject. Examples include: arrival at the clinical facility, completion of triaging, performance of a medical test, analysis of results by a clinician, consultation with clinician, departure from clinical facility and so on.

A target medical event is a medical event under investigation.

The timestamp for the target medical event is established or defined using one or more relevant timestamps associated with the target medical event. Relevant timestamps are those that relate to a medical event or are otherwise associated with the target medical event. For instance, one relevant timestamp would be a manually recorded timestamp of the target medical event. Another relevant timestamp would be an automatically recorded timestamp by a machine performing a medical test. Other suitable relevant timestamps will become clear from the remainder of this disclosure.

The approach proposes to process the relevant timestamp(s) associated with a particular medical event to establish or define the timestamp for the target medical event. In particular, a rule-based technique can be used to process the relevant timestamp(s) to define the timestamp for the target medical event. The timestamp can be recorded in a memory or storage, e.g., in an electronic medical record, and may overwrite an existing timestamp for the target medical event in the electronic medical record.

The relevant timestamp(s) includes at least one location-derived timestamp. A location-derived timestamp identifies a time at which a relevant entity (for the target medical event) is at or near one or more potential locations for the target medical event. It has been recognized that the presence of certain entities (e.g., the subject of the target medical events) is essential for a medical event to take place, such that any location-derived timestamp can be used to define an earliest possible time at which the target medical event took place.

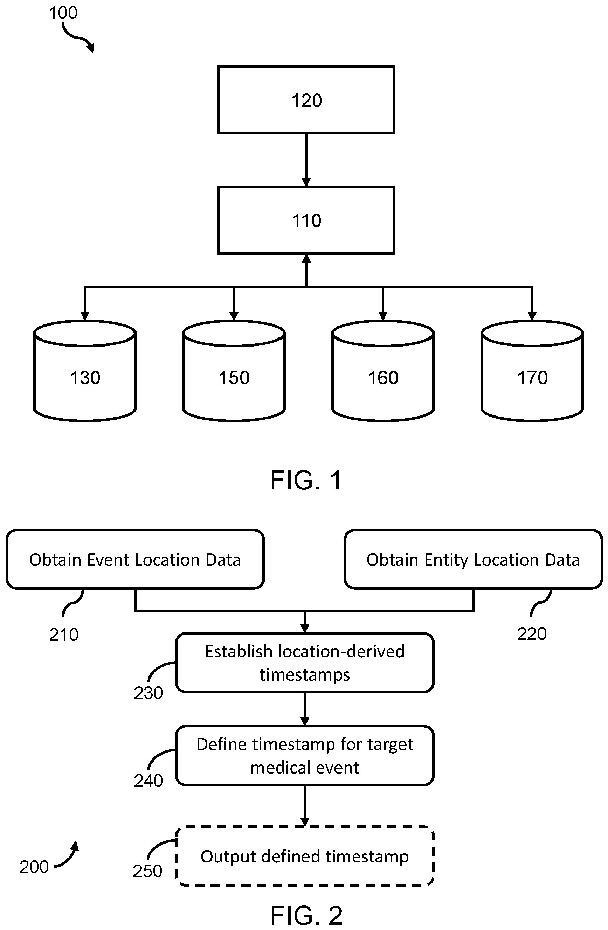

Figure 1 conceptually illustrates an approach according to an embodiment. Relevant timestamps are obtained (e.g., from respective timestamp generators), and are processed by a processing system 110. The processing system 110 applies a rule-based technique to establish or define a timestamp for a target medical event.

As previously mentioned, one important set of one or more relevant timestamps includes a set of one or more location-derived timestamps. The location-derived timestamp(s) may be obtained using information provided by a real-time location tracking system 120 that tracks the location of one or more relevant entities.

In particular, the processing system 110 is configured to obtain event location data identifying one or more potential locations for the target medical event. Different target medical events will have different potential locations where the medical event can or should take place. For instance, a surgical event may take place in any one of one or more surgical rooms. A consultation event may take place in any one of one or more consultation rooms or wards. Other examples will be readily apparent to the skilled person.

Thus, a potential location is a location of a clinical environment in which the medical event is able to take place or is expected to take place.

The event location data may be retrieved, for instance, from an event database 130 that stores an event dataset that maps or correlates (target) medical events to/with one or more potential locations. Thus, the event dataset may identify the potential location(s) for a target medical event.

The processing system 110 is also configured to obtain entity location data, e.g., from the real-time location system 120. The entity location data may comprise, for one or more relevant entities, one or more location data entries, each location data entry identifying a location of the relevant entity and a location timestamp. A location timestamp identifies (for the location of the location data entry) a time or time point at which the relevant entity is at said location.

A relevant entity is an entity associated with the target medical event, e.g., an entity that is required for medical event to take place. Suitable examples will be given later in this disclosure.

Approaches for generating entity location data of this sort are well known in the art, e.g., employing known real-time location tracking systems.

As one example, an entity may be associated with a tag which is scanned each time the entity enters (and optionally leaves) a particular location. The time at which the tag is scanned upon entry to the location may act as a location timestamp.

As a working example, as part of a registration process, a tag can be scanned to connect the identifier of the tag to the subject's identifier in a Hospital Information System (HIS). Subsequently, the tag can be placed on the wrist of the subject. The tag comprises an infra-red (IR) reader, that reads the unique IR signals transmitted by IR beacons in the hospital. In this way, location information of the subject is obtained as the subject moves around the hospital. During a discharge process, the tag can be removed from the subject's wrist and dropped into a "drop box", an enclosed case with an opening allowing patient tags to be dropped into it. The drop box is connected to the Hospital Information System and logs the exact time at which the tag is dropped.

As another example, an image/video analysis technique may be used to analyze image or video data of different locations to identify the presence of the relevant entity in said location(s). A location timestamp can be derived from the metadata of the image/video data when the relevant entity is identified in the location. Approaches for analyzing image/video data are well established in the art, e.g., employing machine-learning algorithms or the like to perform image classification and/or segmentation.

As yet another example, an entity may be associated with a beacon (e.g., a mobile device such as a smart phone) whose location can be tracked in real-time using triangulation and/or trilateration techniques. Approaches for performing this process in various environments are established in the art.

The processing system 110 is also configured to process the event location data and the entity location data to establish one or more location-derived timestamps, each location-derived timestamp identifying a time at which a respective one of the one or more relevant entities is at or near the one or more potential locations for the target medical event identified by the event location data.

This can be trivially performed by identifying the one or more potential locations for the target medical event from the event location data. The entity location data is then parsed or processed to identify the location timestamps for the one or more potential locations. The identified location timestamps subsequently act as the location-derived timestamp(s).

In the context of the present disclosure, "near" may indicate that the distance between the potential location(s) and the relevant entity is less than a predetermined value, e.g., less than 5m, e.g., less than 10m or less than 20m. The predetermined value may depend upon the type of the potential location and/or the environment.

The processing system 110 then processes at least the one or more location-derived timestamps using a rule-based technique to define the timestamp for the target medical event. Thus, the one or more location-derived timestamps form at least part of the relevant timestamp(s) processed by the processing system to define the timestamp for the target medical event.

It will be appreciated that the precise rule-based technique, e.g., set of one or more rules, applied by the processing system 110 to define the timestamp for the target medical event will depend upon the available relevant timestamp(s) for the target medical event, and may be dependent upon the target medical event itself.

It has previously been identified that the location-derived timestamp(s) define when a relevant entity is at or near one or more potential locations for the medical event. Suitable examples of relevant entities are hereafter provided.

The relevant entity may be a human or non-human entity associated with the target medical event. Entities may thereby include equipment or apparatus used for performing or recording the target medical event.

The relevant entity may be the subject of the target medical event, i.e., the subject to whom the medical event occurs. It is recognized that the occurrence of many target medical events necessitates the presence of the subject. A location-derived timestamp for the subject may be labelled a subject location-derived timestamp.

The relevant entity may be a clinician for the target medical event. Different medical events may require different (types of) clinicians. For instance, a surgical procedure will require a surgeon whereas a triaging procedure requires a nurse or other practitioner.

The relevant entity may be a non-clinical personnel of the medical environment, e.g., a porter. Some medical events require non-clinical personnel, for example, to transport the subject to an event location or to move relevant medical equipment to the subject, such that the location of relevant non-clinical personnel may be useful in identifying the earliest possible time that a medical event occurs.

The relevant entity may be a non-clinical associate of the subject (e.g., a family member, guardian, friend etc.). Some clinical events, e.g., consultations, may require such other human entities to be present to take place.

A timestamp for a clinician, non-clinical personnel or non-clinical associate may be labelled a non-subject individual location-derived timestamp.

A non-human example of a relevant entity has been previously described. In particular, the relevant entity may be a piece of equipment for the target medical event. A timestamp for a piece of equipment may be labelled an equipment location-derived timestamp.

The processing system 110 may be required to identify the relevant entity or entities for the target medical event. This can be performed through the use of a relevant entity database 150. The relevant entity database may store a dataset that maps or correlates medical events to relevant entities. The target medical event can be identified, and mapped to the relevant entity or entities.

The content of the dataset stored by the relevant entity database may be defined, e.g., by a clinician, using medical literature and/or guidance and so on.

Thus, the processing system may be configured to obtain relevant entity data identifying, for a plurality of medical events, one or more entities associated with each medical event; and process the relevant entity data to identify, for the target medical event, the one or more relevant entities for the target medical event.

It has been previously explained how the processing system processes at least the one or more location-derived timestamps using a rule-based technique to define the timestamp for the target medical event.

As a simple example, it is recognized the timestamp for a target medical event cannot be earlier than a time at which the subject of the target medical event is at the one or more potential locations. Thus, a rule for a rule-based technique may be one that the timestamp for the target medical event can be no earlier than the earliest location-derived timestamp, e.g., indicating a first known time at which the subject of the target medical event is at the one or more potential locations.

In one embodiment of this example, the timestamp for a target medical event may be set to be equal to the earliest location-derived timestamp for the target medical event.

As another example, the timestamp for a target medical event can be defined to be a time no earlier than when all relevant entities are (e.g., first) at the one or more potential locations for the target medical event. Thus, a rule for a rule-based technique may be one that the timestamp for the target medical event can be no earlier than the earliest location-derived timestamp that indicates the last relevant entity to join all other relevant entities at or near a same location within the one or more potential locations.

In one embodiment of this example, the timestamp for a target medical event may be set to be equal to this location-derived timestamp.

Previous examples provide approaches for defining the timestamp for the target medical event from the location-derived timestamps alone. However, one or more other relevant timestamps may be processed by the processing system to define or establish the timestamp.

In one example, the processing system is configured to receive one or more HIS timestamps. An HIS timestamp is a timestamp recorded by a health information system. Examples of HIS timestamps include a manually recorded timestamp (e.g., recorded in an electronic medical record of a subject) and an automatically recorded timestamp, e.g., by a system used to perform or in performing a medical event.

The rule-based technique used by the processing system 110 may include one or more rules that defines which of the relevant timestamps to use. The rule may perform one or more comparisons between the relevant timestamps to define which timestamp to use.

For instance, one rule could say to take a manually recorded timestamp if available, and a location-derived timestamp otherwise.

As another example, another possible rule could say to take the earliest of the relevant timestamps as the timestamp for the target medical event.

The above-described embodiments make use of relevant timestamps that record a single target medical event to define or establish the timestamp for the target medical event (e.g., the "true" timestamp).

However, timestamps for other medical events could be used to determine or define the timestamp for a target medical event. In particular, if a workflow or care pathway for a subject is known, this information (together with the timestamps of other medical events) can be used to define the timestamp for the target medical event.

Thus, the processing system 110 may be configured to obtain event workflow data that defines an expected sequence for a plurality of medical events for the subject, the plurality of medical events including at least the target medical event. Thus, the workflow data may identify an expected sequence of medical events for the subject.

The processing system 110 may obtain the event workflow data, for instance, by consulting or communicating with a workflow database 160 storing a workflow dataset.

It is recognized that there are established workflows or care pathways for treating or assessing a subject, e.g., based on their condition or pathology. The workflow from workflow database 160 that applies to the subject may be obtained from information in an electronic medical record, e.g., stored by an EMR database 170, pertaining to the subject.

In other words, an electronic medical record (e.g., defining a medical history of the subject) of the subject may be processed to identify the workflow or care pathway for the subject, i.e., the expected sequence of a plurality of medical events for the subject.

The processing system may also obtain workflow timestamp data defining at least one timestamp for each of the plurality of medical events in the event workflow data. In this way, at least one timestamp is identified for each event in the sequence of events. Any previous example of a timestamp can be employed (e.g., a location-derived timestamp, a manually recorded timestamp and/or an automatically recorded timestamp).

Processing, using a rule-based technique, may further process the event workflow data and workflow timestamp data to define the timestamp for the target medical event.

This approach recognizes that the timestamps for events located elsewhere in a sequence of events (including the target medical event) can define possible bounds for the timestamp of the target medical event. For instance, a timestamp for a medical event later in the sequence may define a latest possible time that the target medical event could have been performed.

To improve the accuracy of using other timestamps, in some examples, only automatically recorded timestamps of other medical events (in the sequence) are used to define the timestamp for the target medical event. Automatically recorded timestamps benefit from improved accuracy.

Thus, the sequence of events may comprise the target medical event and events for which an automatically recorded timestamp is available, i.e. automatically recordable events. Similarly, the workflow timestamp data may comprise, for each of one or more automatically recordable events in the plurality of events, an automatically recorded timestamp for the automatically recordable event.

Processing, using a rule-based technique, may comprise defining the timestamp for the target medical event to be no later than the earliest of any automatically recorded timestamp for any medical event later in the expected sequence, defined by the event workflow data, than the target medical event.

Similarly, processing, using a rule-based technique, may comprise defining the timestamp for the target medical event to be no earlier than the latest of any automatically recorded timestamp for any medical event earlier in the expected sequence, defined by the event workflow data, than the target medical event.

In this way, a more accurate determination of timestamp for the target medical event can be achieved.

Previous embodiments have indicated various approaches for using relevant timestamps to identify the earliest possible and/or latest possible time at which the target medical event can occur. This can make use, for example, of location-derived timestamps for the target medical event or automatically-derived timestamps for other medical events in a sequence of medical events.

In this way, it will be understood that the rule-based technique may use the relevant timestamps for a medical event to define the bounds for the (true or later defined) timestamp for the medical event. In particular, the relevant timestamps may be processed to identify the earliest possible time and/or the latest possible time at which the medical event can occur, and use this information to select a timestamp for medical event.

For instance, location-derived timestamps are able to define a bound for an earliest possible time at which the medical event occurs. Location-derived timestamps may also be usable to define a bound for latest possible time at which the medical event occurs (e.g., by tracking when the one or more relevant entities leave the expected location(s)).

Similarly, an automatically generated timestamp for a medical event that occurs sequentially or temporarily before the target medical event may define a bound for an earliest possible time that the target medical event occurs. An automatically generated timestamp for a medical event that occurs sequentially or temporarily after the target medical event may define a bound for a latest possible time that the target medical event occurs.

This information and understanding can be implemented in a set of rules for resolving conflicts between different relevant timestamps for the subject.

The set of rules used to define the timestamp for the target medical event may include a rule that an automatically-generated timestamp (if available) for the target medical event is selected as the timestamp, unless this contradicts with the bounds for the earliest/latest possible time for the target medical event defined by any other relevant timestamp for the target medical event.

The set of rules used to define the timestamp for the target medical event may include a rule that a manually-generated timestamp (if no automatically-generated timestamp is available) for the target medical event is selected as the timestamp, unless this contradicts with the bounds for the earliest/latest possible time for the target medical event defined by any other relevant timestamp for the target medical event.

If no timestamp is selectable from this information, then the rule-based technique may select one of the bounds to act as the timestamp for the target medical event. The selected bound may depend, for instance, on the type of the target medical event, such that the "worse case" timestamp may be selected. For instance, if the target medical event is a time of arrival at a clinical environment, the selected bound may be the earliest bound (e.g., as increased time since arrival is generally seen as a negative characteristic). If the target medical event is taking of a restricted medication, the selected bound may be the latest bound (e.g., to minimize risk of potential overdosing).

Other suitable reasons and justifications for selecting timestamps for various medical events will be apparent to the skilled person based on at least this understanding, and could be included in the rule-based technique.

The processing system 110 may be configured to output the timestamp for the target medical event.

In a particularly advantageous embodiment, the processing system is configured to write or store the timestamp for the target medical event to/in an electronic medical record for the subject, e.g., stored by an EMR database 170. This process may, for instance, overwrite an existing or recorded timestamp (e.g., a manually recorded timestamp) for the target medical event in the electronic medical record. If no existing or recorded timestamp is present in the electronic medical record, this step may comprise setting or writing the recorded timestamp to be equal to the defined timestamp. This ensures that later consultation of the medical record, e.g., for the purposes of assessing the condition of the patient and so on, refers to a more accurate timestamp.

In other words, outputting the defined timestamp for the target medical event may comprise setting and/or modifying a recorded timestamp for the target medical event stored by an electronic medical record to align with (e.g., have the same value as) the defined timestamp for the target medical event.

In some examples, the processing system 110 is configured to output the timestamp to a further processing system, e.g., for further analysis or processing. This can be performed in addition to or instead of modifying/setting the timestamp for the target medical event in the electronic medical record.

For improved understanding, Figure 2 illustrates a method 200 for defining a timestamp for a target medical event for a subject. The method 200 is a computer-implemented method, i.e., a method performed by a computer or processing system.

The method 200 comprises a step 210 of obtaining event location data identifying one or more potential locations for the target medical event.

The method 200 also comprises a step 220 of obtaining entity location data comprising, for one or more relevant entities, one or more location data entries, each location data entry identifying a location of the relevant entity and a location timestamp, wherein each relevant entity is an entity associated with the target medical event.

The method 200 also comprises a step 230 of processing the event location data and the entity location data to establish or define one or more location-derived timestamps, each location-derived timestamp identifying a time at which a respective one of the one or more relevant entities is at or near one of the one or more potential locations for the target medical event identified by the event location data.

The method 200 also comprises a step 240 of processing at least the one or more location-derived timestamps using a rule-based technique to define the timestamp for the target medical event.

The method may comprise a step 250 of outputting the timestamp for the target medical event.

In a particularly advantageous embodiment, step 250 comprises writing the timestamp for the target medical event to an electronic medical record for the subject. This step may, for instance, overwrite an existing timestamp (e.g., a manually recorded timestamp) for the target medical event in the electronic medical record. This ensures that later consultation of the medical record, e.g., for the purposes of assessing the condition of the patient and so on, refers to a more accurate timestamp.

Thus, step 250 may comprise setting and/or modifying a recorded timestamp for the target medical event stored by an electronic medical record to align with the defined timestamp for the target medical event.

In some examples, step 250 comprises outputting the timestamp to a further processing system, e.g., for further analysis or processing.

It has previously been explained how the set of rules applied (in step 240) may depend upon the target medical event itself, i.e., the type of the target medical event. The set of rules may be represented by a flowchart that identifies the procedure taken to identify a value for the timestamp of a target medical event.

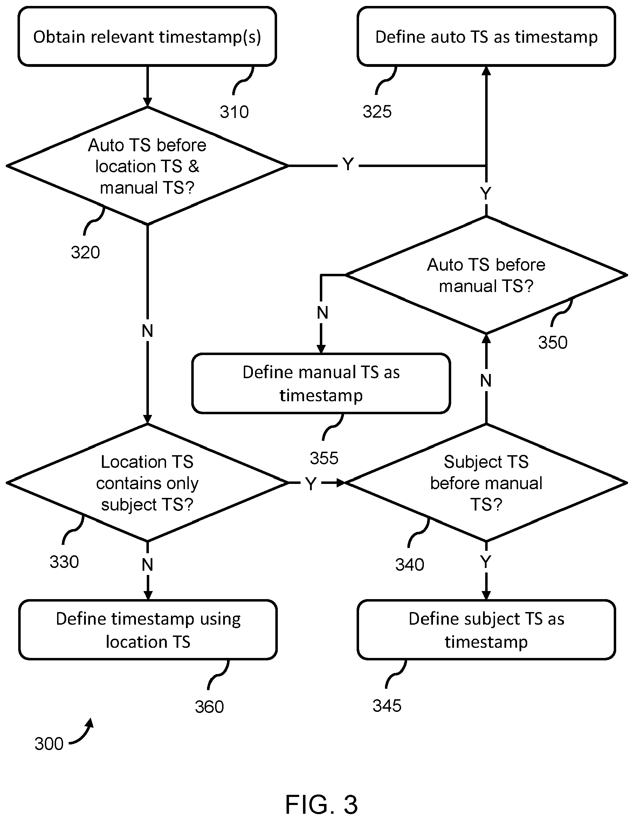

Figure 3 illustrates one example method 300 for determining a timestamp for a first target medical event, being an event of "arrival at the clinical environment". The acronym TS used in Figure 3 stands for timestamp or timestamps.

In step 310, relevant timestamps for the first target medical event are obtained. The relevant timestamps here include one or more location derived timestamps, a manually recorded timestamp (for the target medical event) and (if present) the earliest of any automatically recorded timestamps for a medical event that occurs later in the sequence of medical events than the target medical event (the "earliest automatically recorded timestamp"). For instance, the automatically recorded timestamp may be a time at which a CT scan was performed on the subject of the target medical event.

In step 320, if present, it is determined whether or not the earliest of any automatically recorded timestamp(s) is temporally before the location-derived timestamp(s) and the manually recorded timestamp.

In response to a positive determination being made in step 320, the method 300 moves to step 325 of defining the automatically recorded timestamp as the timestamp for the target medical event. This recognizes that it is not possible for the arrival of the patient (i.e., the target medical event in this scenario) to occur after the automatically recorded timestamp was made. Thus, a timestamp that is closer to the true timestamp (e.g., a more accurate timestamp) is defined for the target medical event.

In response to a negative determination being made in step 320, the method 300 moves to step 330. Step 330 comprises determining whether the one or more location-derived timestamps includes only a subject location-derived timestamp.

The method may move directly to step 330 if no automatically recorded timestamps are available.

In response to a positive determination being made in step 330, the method may perform a step 340 of determining whether the subject location-derived timestamp is before the manually recorded timestamp.

In response to a positive determination being made in step 340, the method moves to a step 345 of defining the subject location-derived timestamp as the timestamp for the target medical event.

In response to a negative determination being made in step 340, the method moves to a step 350 of determining whether the automatically recorded timestamp (if present) is before the manually recorded timestamp. In response to a positive determination being made in step 350, the method performs step 325. Otherwise, a step 355 of defining the manually recorded timestamp as the timestamp for the target medical event is performed.

In response to a negative determination being made in step 330, the method moves to a step 360 of defining the timestamp for the target medical event using the location-derived timestamps. For instance, step 360 may comprise defining the timestamp to be equal to an earliest timestamp at which all relevant entities (as tracked using the location-derived timestamps) are at or near a same location within the one or more potential locations for the target medical event.

The method 300 provides one example flowchart or set of rules that could be applied to determine a timestamp for a first medical event. The skilled person will appreciate that other similar flowcharts or rule sets can be employed for other medical events.

The method 300 may be appropriately adapted based on availability of timestamps or other data.

The skilled person would be readily capable of developing a processing system for carrying out any herein described method. Thus, each step of the flow chart may represent a different action performed by a processing system, and may be performed by a respective module of the processing system.

Embodiments therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. A computer-implemented method for defining a timestamp for a target medical event for a subject, the computer-implemented method comprising:
obtaining event location data identifying one or more potential locations for the target medical event;
obtaining entity location data comprising, for one or more relevant entities, one or more location data entries, each location data entry identifying a location of the relevant entity and a location timestamp, wherein each relevant entity is an entity associated with the target medical event;
processing the event location data and the entity location data to establish one or more location-derived timestamps, each location-derived timestamp identifying a time at which a respective one of the one or more relevant entities is at or near one of the one or more potential locations for the target medical event identified by the event location data; and
processing at least the one or more location-derived timestamps using a rule-based technique to define the timestamp for the target medical event.

2. The computer-implemented method of claim 1, wherein the one or more relevant entities includes the subject of the target medical event, and the one or more location-derived timestamps comprises a subject location-derived timestamp indicating a time at which the subject is at or near the one or more potential locations for the target medical event identified by the event location data.

3. The computer-implemented method of claim 2, wherein the step of processing, using a rule-based technique, comprises defining the timestamp for the target medical event to be no earlier than the subject location-derived timestamp.

4. The computer-implemented method of any of claims 1 to 3, wherein:
the one or more relevant entities includes one or more clinicians or non-subject individuals involved in the medical event; and
the one or more location-derived timestamps comprises one or more non-subject individual location-derived timestamps, each indicating a time at which a respective clinician or non-subject individual is at or near the one or more potential locations for the target medical event identified by the event location data.

5. The computer-implemented method of claim 4, wherein the step of processing, using a rule-based technique, comprises defining the timestamp for the target medical event to be no earlier than any of the one or more non-subject individual location-derived timestamps.

6. The computer-implemented method of any of claims 1 to 5, wherein:
the one or more relevant entities includes one or more pieces of equipment for the target medical event; and
the one or more location-derived timestamps comprises one or more equipment location-derived timestamps, each indicating a time at which a respective piece of equipment is at or near the one or more potential locations for the target medical event identified by the event location data.

7. The computer-implemented method of claim 6, wherein the step of processing, using a rule-based technique, comprises defining the timestamp for the target medical event to be no earlier than any of the one or more non-subject individual location-derived timestamps.

8. The computer-implemented method of any of claims 1 to 7, further comprising:
obtaining relevant entity data identifying, for a plurality of medical events, one or more entities associated with each medical event; and
processing the relevant entity data to identify, for the target medical event, the one or more relevant entities for the target medical event.

9. The computer-implemented method of any of claims 1 to 8, further comprising, obtaining, if available, a manually recorded timestamp for the target medical event, wherein,
the step of processing, using a rule-based technique, further processes at least the manually recorded timestamp, if available, to define the timestamp for the target medical event.

10. The computer-implemented method of any of claims 1 to 9, further comprising:
obtaining event workflow data defining the expected sequence for a plurality of medical events for the subject, the plurality of medical events including at least the target medical event; and
obtaining workflow timestamp data defining at least one timestamp for each of the plurality of medical events in the event workflow data; and
wherein the step of processing, using a rule-based technique, further processes the event workflow data and workflow timestamp data to define the timestamp for the target medical event.

11. The computer-implemented method of claim 10, wherein:
the workflow timestamp data comprises, for each of one or more automatically recordable events in the plurality of events, an automatically recorded timestamp for the automatically recordable event; and
the step of processing, using a rule-based technique, comprises defining the timestamp for the target medical event to be no later than the automatically recorded timestamp for any medical event later in the expected sequence, defined by the event workflow data, than the target medical event.

12. The computer-implemented method of any of claims 1 to 11, further comprising a step of outputting the defined timestamp for the target medical event.

13. The computer-implemented method of claim 12, wherein outputting the defined timestamp for the target medical event comprises setting and/or modifying a recorded timestamp for the target medical event stored by an electronic medical record to align with the defined timestamp for the target medical event.

14. A computer program product comprising computer program code means which, when executed on a computing device having a processing system, cause the processing system to perform all of the steps of the method according to any of claims 1 to 13.

15. A processing system for defining a timestamp for a target medical event for a subject, the processing system being configured to:
obtain event location data identifying one or more potential locations for the target medical event;
obtain entity location data comprising, for one or more relevant entities, one or more location data entries, each location data entry identifying a location of the relevant entity and a location timestamp, wherein each relevant entity is an entity associated with the target medical event;
process the event location data and the entity location data to establish one or more location-derived timestamps, each location-derived timestamp identifying a time at which a respective one of the one or more relevant entities is at or near one of the one or more potential locations for the target medical event identified by the event location data; and
process at least the one or more location-derived timestamps using a rule-based technique to define the timestamp for the target medical event.
